# Europäisches Patentamt
# European Patent Office
# Office européen des brevets

(19)

(11) Numéro de publication: **0 039 415**
**B1**

(12)

# FASCICULE DE BREVET EUROPEEN

(45) Date de publication du fascicule du brevet:
**23.03.83**

(51) Int. Cl.³: **C 12 N 1/06**

(21) Numéro de dépôt: **81102552.7**

(22) Date de dépôt: **04.04.81**

(54) **Procédé de fabrication d'un extrait de levure.**

(30) Priorité: **02.05.80 CH 3430/80**

(43) Date de publication de la demande:
**11.11.81 Bulletin 81/45**

(45) Mention de la délivrance du brevet:
**23.03.83 Bulletin 83/12**

(84) Etats contractants désignés:
**AT BE DE FR IT NL SE**

(56) Documents cités:
**FR-A-1 439 073**
**FR-A-1 568 432**
**FR-A-2 225 515**
**FR-A-2 367 433**
**FR-A-2 438 088**
**GB-A-1 432 522**
**US-A-2 095 300**

(73) Titulaire: **SOCIETE DES PRODUITS NESTLE S.A., Case postale 353, CH-1800 Vevey (CH)**

(72) Inventeur: **Rehacek, Josef, Roselière 13, CH-1400 Yverdon (CH)**
Inventeur: **Dasek, Jaroslav, Rue du Levant 2A, CH-1400 Yverdon (CH)**

BUNDESDRUCKEREI BERLIN

## Procédé de fabrication d'un extrait de levure

La présente invention a pour objet un procédé de fabrication d'un extrait de levure, dans lequel on prépare une suspension de cellules de levure, on désintègre mécaniquement les cellules, on les livre à l'autolyse et l'on renforce l'autolyse par addition d'enzymes protéolytiques.

La production industrielle des extraits de levure repose sur divers procédés connus tels que la plasmolyse, l'autolyse, la combinaison de la plasmolyse et de l'autolyse ou même leur renforcement par une addition d'enzymes protéolytiques.

Dans la plasmolyse, le contenu cellulaire est libéré de la cellule. Il franchit les membranes cellulaires endommagées soit par choc thermique, soit sous l'effet de la concentration hypertonique de sels ou de sucres dans une suspension concentrée de levures, soit encore sous l'effet de certains solvants. L'extrait ainsi obtenu présente divers défauts qui sont la faible dégradation des protéines, la faible teneur en vitamines et le goût peu intéressant.

Dans l'autolyse, les constituants protoplasmiques sont dégradés par les enzymes mêmes de la levure. Durant l'autolyse, les membranes, voire les parois cellulaires sont également attaquées, ce qui entraîne la libération du protoplasme autolysé. Le contrôle des mécanismes de l'autolyse a été poussé assez loin et il est possible d'influencer le rendement du procédé et les qualités organoleptiques du produit final. Cependant, le gros défaut du procédé réside dans les risques inhérents à sa longue durée, notamment le risque de contamination de la suspension en cours d'autolyse qui représente un milieu très favorable à la prolifération de microorganismes. Pour pallier ce défaut, l'usage de divers agents antimicrobiens a été décrit, notamment des solvants organiques non polaires, chloroforme, toluène ou trichloréthylène, à des concentrations allant jusqu'à 5%, l'éthanol ou le chlorure de sodium, par exemple. Mais l'usage de ces agents ne va pas sans inconvénients et, si l'on considère le dernier exemple, on constate que la protection de l'autolyse par le chlorure de sodium a haute concentration provoque l'inhibition partielle des enzymes autolytiques et entraîne un prolongement de la durée de la lyse ou un fort abaissement du rendement, sans compter l'effet de corrosion des installations. De plus, le produit final présente un forte teneur en sel.

Pour les procédés connus basés sur la combinaison de la plasmolyse et de l'autolyse, la plasmolyse est le plus souvent provoquée par l'adjonction d'une quantité élevée de sel dans une suspension concentrée de levures. Cette dernière présente généralement une teneur en matière sèche supérieure à 20% et le sel y est ajouté à raison d'au moins 2 parties pour 10 parties de poids sec de levure. Cette quantité de sel n'assure cependant pas encore l'asepsie du processus et une addition d'éthanol jusqu'à 9% du volume total a été proposée. Le temps d'autolyse dépasse 60 h dans ces conditions et même si la suspension finale est passée à l'autoclave pour augmenter le rendement, celui-ci ne dépasse pas 60%. On connaît même un procédé de ce genre basé sur l'endommagement des membranes cellulaires par choc thermique réalisé par séchage par atomisation de la suspension. L'autolyse des levures remises en suspension dans l'eau est réalisée alors pendant 24 à 48 h à 45°C sans qu'aucune protection microbienne ne soit prévue:

Enfin, en ce qui concerne la combinaison de la plasmolyse et de l'autolyse renforcée par l'addition d'enzymes protéolytiques, divers procédés de ce type ont été proposés qui ont en commun l'avantage d'être plus aisément contrôlables et de raccourcir le temps d'incubation nécessaire. Cependant, les rendements obtenus ne dépassent guère 55%.

FR-A-2 225 515 décrit un procédé de lyse de parois de cellules de levure, dans lequel la lyse enzymatique de ces parois est favorisée soit par un prétraitement des levures dans une solution de sulfite, soit par la réalisation de la lyse dans une solution »activante« contenant du sulfite et/ou un sel minéral. Dans le cas du prétraitement dans la solution de sulfite, le sulfite est éliminé par lavage des levures à l'eau avant la lyse. Dans le cas de la lyse dans un milieu réactionnel au sulfite et/ou sel minéral, ceux-ci restent en solution.

La présente invention est le fruit de la recherche d'un procédé simple et efficace qui ne présente pas les défauts ci-dessus des procédés connus, et qui permette de produire en conditions parfaitement aseptiques et avec un grand rendement un extrait de haute valeur nutritive et de qualités organoleptiques irréprochables.

Le procédé de fabrication d'un extrait de levure selon la présente invention, dans lequel on prépare une suspension de cellules de levures, on désintègre mécaniquement les cellules, on les livre à l'autolyse et l'on renforce l'autolyse par addition d'enzymes protéolytiques, est caractérisé par le fait que l'on ajoute un sulfite à la suspension de levure avant ou après la désintégration mécanique, on sépare les insolubles de la suspension hydrolysée, on ajoute un composé du calcium au surnageant et l'on clarifie le surnageant.

Grâce au procédé selon la présente invention, on est en mesure de produire un extrait très pur, en un temps très court, dans des conditions particulièrement bien contrôlables et que l'on peut aisément varier à volonté et avec un rendement inégalé jusque-là. On a constaté en particulier que, par l'addition de sulfite avant ou durant l'autolyse, on obtient non seulement une activation des enzymes protéolytiques et une protection antimicrobienne sûre, mais ensuite également un effet de précipitation inédit et précieux dans le surnageant de la suspension autolysée centrifugée, lorsqu'on y ajoute un composé du calcium, qui permet une clarification de ce surnageant. Ce dernier est toujours rendu trouble par la présence de particules que l'on n'arrive pas à séparer. On pense que celles-ci sont fixées et entraînées dans la précipitation du composé insoluble qui se forme entre le sulfite et le composé du calcium.

Un autre avantage du présent procédé réside dans le fait que le résidu non soluble obtenu lors de la centrifugation n'est absolument pas contaminé et que l'on peut parfaitement l'utiliser directement dans la fabrication de produits alimentaires.

Enfin, le présent procédé permet la fabrication d'extraits de levure pauvres en sel, dans lesquels la concentration de chlorure de sodium ne dépasse pas 6%, soit trois fois moins que dans les extraits usuels. Un tel produit présente un grant intérêt au poit de vue diététique.

Pour mettre en oeuvre le présent procédé, on peut utiliser comme matière première les levures usuelles utilisées pour la fabrication d'extraits de levure, notamment Saccharomyces cerevisiae, levures de boulangerie et levures de brasserie, et Candida utilis. On en prépare de préférence une suspension présentant une teneur en matière sèche de 10 à 25%.

On peut réaliser la désintégration mécanique avec tout moulin, homogénéisateur ou appareil qui travaille sur le principe de l'expansion brutale capable de libérer le protoplasme avec les endo-enzymes dans des conditions où ces dernières conservent leur pleine activité. On peut notamment utiliser à cet effet des moulins à billes, des homogénéisateurs par laminage de fluide sous pression ou des désintégrateurs à ultrasons, par exemple.

Selon un mode d'exécution préféré, on ajuste le pH de la suspension à une valeur comprise entre 4,4 et 6,8 avant la désintégration et à une valeur »neutre« de 6,5 à 7 après la désintégration, on ajoute au moins une enzyme protéolytique active à pH neutre, on incube sous légère agitation durant 15 à 22 h à pH neutre et l'on ajoute en plusieurs fois durant l'incubation un total de 300 à 2000 ppm d'un sulfite et un total de 300 à 2000 ppm de formaldéhyde.

Par sulfite, il faut entendre dans le présent exposé tout composé chimique alimentaire capable de libérer du dioxyde de soufre, et notamment les sulfite, disulfite et métabisulfite de sodium et de potassium, le dioxyde de soufre lui-même entrant en considération.

Selon un autre mode d'exécution préféré, on ajuste le pH de la suspension à une valeur comprise entre 3,0 et 5,5 et l'on ajoute de 200 à 2000 ppm d'un sulfite avant la désintégration. On peut même laisser agir 30 à 60 min sous léfère agitation à température ambiante avant la désintégration. Au cours de la désintégration, le pH peut monter spontanément à 5,1-6,0. On ajoute au moins une enzyme protéolytique active à pH acide et l'on incube sous légère agitation durant 15 à 22 h à pH acide.

Pour réaliser les ajustements de pH, on peut utiliser tout acide ou base alimentaire. On utilise de préférence l'acide chlorhydrique et l'hydroxyde de sodium. Le choix de l'enzyme protéolytique est fonction de son activité, des températures et pH nécessaires et de son prix en regard des qualités organoleptiques des produits de l'autolyse renforcée. On peut obtenir de très bons résultats dans les deux formes d'exécutions ci-dessus avec la papaïne, à raison d'au moins 0,3 g de papaïne par litre de suspension et avec des températures d'incubation de 45 à 65°C. Mais le présent procédé n'est pas limité à l'usage de cette enzyme et d'autres peuvent très bien convenir, notamment la trypsine et d'autres protéases d'origines diverses ainsi que leurs mélanges, à des pH et à des températures d'incubation adéquats.

Après l'autolyse renforcée par addition d'enzyme, on peut inactiver les enzymes par chauffage. On chauffe de préférence la suspension à 90 – 95°C durant 35 à 70 min. Ceci assure non seulement l'inactivation des enzymes, mais également une certaine coagulation des insolubles. Ceux-ci peuvent alors être séparés par filtration ou centrifugation par exemple. On préfère réaliser la séparation par centrifugation à chaud, à 60 – 80°C.

Après la séparation des insolubles, il reste dans le surnageant de très fines particules qui provoquent une turbidité. Il y reste également l'intégralité du sulfite. On ajoute alors à la suspension un composé du calcium que l'on choisit de préférence parmi les composés solubles tels que le chlorure, le gluconate ou l'hydroxyde de calcium. On l'ajoute de préférence en quantité équimolaire du sulfite et à un pH de 6,5 à 7,5. On obtient un précipité que l'on sépare. On peut effectuer cette clarification par filtration ou par centrifugation par exemple.

La solution obtenue peut être alors concentrée par évaporation sous vide par exemple pour obtenir une pâte ou séchée par atomisation par exemple pour obtenir une poudre. Le produit que le présent procédé permet ainsi d'obtenir représente jusqu'à plus de 70% en poids de matière sèche de la levure de départ. Un tel rendement est tout à fait remarquable.

Le produit obtenu par le présent procédé, qui présente des qualités organoleptiques excellentes et contrôlables et une composition chimique parfaitement reproductible même avec différentes levures au départ, peut être utilisé comme supplément ou condiment pour soupes, bouillons, sauces, viandes, ragoûts, poissons, légumes, amuse-bouches, pains et biscuits, comme additif riche en vitamines pour confiserie, chocolat, bonbons, produits laitiers, notamment fromages, et produits diététiques, et comme composant essentiel des milieux de culture en fermentation industrielle, par exemple.

Les exemples ci-après sont donnés à titre d'illustration. Les pourcentages y sont exprimés en poids.

### Exemple 1

On prépare 10 l d'une suspension de levures Candida utilis présentant une teneur en matière sèche de 16%. On ajuste son pH à 3,5 par addition d'acide chlorhydrique à 15%. On ajoute 100 ml d'une solution à 10% de sulfite de sodium et l'on brasse doucement durant 40 min à température ambiante. On soumet la suspension à une désintégration en continu dans un broyeur à billes horizontal du type WAB-KDL-Pilot. On recueille une suspension de cellules broyées à 86 – 90% présentant un pH de 5,5 dans une cuve à double manteau.

On ajoute de la papaïne »cristalase A« de la maison ABM, à raison de 3 g par litre de suspension. On incube durant 20 h à 55°C. On inactive les enzymes par chauffage à 90°C durant 1 h. On centrifuge la suspension à chaud, à 60 – 65°C, avec une centrifugeuse à débourbage automatique.

On neutralise le surnageant à un pH de 6,8 à 7 par addition d'hydroxyde de sodium. On ajoute du chlorure de calcium en quantité équimolaire du sulfite de sodium ajouté au début. On sépare le précipité par une deuxième centrifugation à chaud.

On abaisse le pH de la solution à 5,4 par addition d'HCl à 15% et l'on ajoute du chlorure de sodium de manière à en obtenir une concentration de 13% dans une pâte finale à 80% de matière sèche. On concentre la solution par évaporation sous vide à une température de 75 – 80°C jusqu'à une teneur en matière sèche de 70%. On chauffe à 90°C durant 30 min à pression atmosphérique pour provoquer une réaction de Maillard et l'on poursuit l'évaporation jusqu'à une teneur en matière sèche de 80%.

On obtient un extrait de levure présentant la composition suivante:

| | |
|---|---|
| Matière sèche | 81,6% |
| Azote total | 7,2% |
| Azote $\alpha$-aminé libre | 2,0% |
| Matières réductrices | 4,1% |
| Chlorure de sodium | 12,9% |
| Cendres | 17,9% |
| Couleur (extinction à 480 nm d'une solution à 5%) | 1,16 |

La totalité des germes n'excède pas 10 à 20 par gramme de produit.

Le rendement de la fabrication, calculé par rapport au poids sec des levures de départ, est de 71%. Le produit présente un goût très pur et corsé et se prête particulièrement bien à l'usage comme condiment dans les soupes, sauces et autres mets relevés.

### Exemple 2

On procède de la manière décrite à l'exemple 1 jusqu'à la deuxième centrifugation, à l'exception du fait que l'on utilise de la papaïne No. 7135 de la maison Merck, à raison de 1,5 g par litre de suspension, et que l'on incube durant 18 h.

On lave les insolubles obtenus après la première centrifugation. On ajuste leur pH à 7 et on les stérilise à 115°C durant 15 min. On obtient une pâte gélatineuse de texture très fine et de couleur crème que l'on peut utiliser comme additif à faible valeur calorique dans les produits alimentaires et qui présente la composition suivante:

| | |
|---|---|
| Matière sèche | 16% |
| Azote total | 0,6% |
| Glucides totaux (glucosemannose en proportion 1 : 1) | 5,8% |
| Matières grasses | 2,5% |
| Cendres | 1,2% |

On abaisse à 5,2 le pH de la solution obtenue après la deuxième centrifugation. On concentre la solution jusqu'à une teneur en matière sèche de 30% et on la filtre sur charbon actif. On continue l'évaporation sous vide, à température modérée, pour éviter au maximum toute réaction de Maillard. On obtient une pâte d'extrait de levure présentant la composition suivante:

| | |
|---|---|
| Matière sèche | 80% |
| Azote total | 7,8% |
| Azote $\alpha$-aminé libre | 1,7% |
| Chlorue de sodium | 5,8% |
| Cendres | 11,0% |
| Matières grasses | 0,1% |
| Couleur (extinction à 480 nm d'une solution à 5%) | 0,12 |

La pâte présente un goût neutre et se prête particulièrement bien à l'usage comme additif ou supplément vitaminé en confiserie ou dans les produits laitiers par exemple.

### Exemple 3

On prépare un extrait de la manière décrite à l'exemple 1, à l'exception du fait que l'on n'ajoute pas de chlorure de sodium à la solution après la deuxième centrifugation et qu'on la concentre par évaporation sous vide à température modérée, pour éviter autant que possible toute réaction de Maillard, jusqu'à une teneur en matière sèche de 80%. On obtient un extrait de levure de goût neutre et de couleur claire présentant une teneur en chlorure de sodium inférieure à 4,5%.

On utilise cet extrait dans la préparation d'un produit diététique à base d'extrait de malt. A cet effet, on prépare un extrait de malt à 66% de matière sèche présentant la presque totalité de l'activité diastasique et protéolytique du malt de départ. On mélange l'extrait de malt et l'extrait de levure dans des proportions comprises entre 9 : 1 et 7 : 3. On ajuste le pH du mélange à 5,0 avec de l'acide lactique.

Le produit correspondant au mélange à 7 parties d'extrait de malt pour 3 parties d'extrait de levure présente une teneur en matière sèche de 71% dont 60% de sucre, déterminé comme glucose, 9,5% de protéines et 0,9% de NaCl. Son activité de l'eau ne dépasse pas 0,62 à 30°C, niveau auquel toute croissance de microorganismes est complètement inhibée. Ses qualités organoleptiques ne diffèrent pratiquement pas de celles de l'extrait de malt initial.

## Revendications

1. Procédé de fabrication d'un extrait de levure, dans lequel on prépare une suspension de cellules de levure, on désintègre mécaniquement les cellules, on les livre à l'autolyse et l'on renforce l'autolyse par l'addition d'enzymes protéolytiques, caractérisé par le fait que l'on ajoute un sulfite à la suspension de levure avant ou après la désintégration mécanique, on sépare les insolubles de la suspension hydrolysée, on ajoute un composé du calcium au surnageant et l'on clarifie le surnageant.

2. Procédé selon la revendication 1, caractérisé par le fait que l'on prépare une suspension de cellules présentant une teneur en matière sèche de 10 à 25% et un pH de 4,4 à 6,8, et pour réaliser l'autolyse renforcée, après désintégration mécanique des cellules, on ajoute au moins une enzyme protéolytique active à pH neutre, on incube sous légère agitation durant 15 à 22 h à pH neutre et l'on ajoute en plusieurs fois durant l'incubation un total de 300 à 2000 ppm d'un sulfite et un total de 300 à 2000 ppm de formaldéhyde.

3. Procédé selon la revendication 1, caractérisé par le fait que l'on prépare une suspension de cellules présentant une teneur en matière sèche de 10 à 25% et un pH de 3,0 à 5,5, on ajoute de 200 à 2000 ppm d'un sulfite, et pour réaliser l'autolyse renforcée, après désintégration mécanique des cellules, on ajoute au moins une enzyme active à pH acide, et l'on incube sous légère agitation durant 15 à 22 h à pH acide.

4. Procédé selon la revendication 1, caractérisé par le fait qu'après l'autolyse renforcée on chauffe la suspension à 90 – 95°C durant 35 à 70 min et l'on sépare les insolubles par centrifugation à 60 – 80°C.

5. Procédé selon la revendication 1, caractérisé par le fait que l'on ajoute le composé du calcium en quantité équimolaire du sulfite à un pH de 6,5 à 7,5 et l'on clarifie en séparant le précipité ainsi obtenu.

6. Procédé selon la revendication 1, caractérisé par le fait que l'enzyme ajoutée est la papaïne,

on l'ajoute à raison de au moins 0,3 g par litre de suspension et l'on incube à une température de 45 à 65°C.

## Patentansprüche

1. Verfahren zur Herstellung eines Hefeextraktes, in welchem man eine Suspension von Hefezellen bereitet, mechanisch die Zellen desintegriert, sie einer Autolyse überläßt und die Autolyse durch Zugabe von proteolytischen Enzymen verstärkt, dadurch gekennzeichnet, daß man zur Hefesuspension vor oder nach der mechanischen Desintegration ein Sulfit zusetzt, die unlöslichen Teile von der hydrolysierten Suspension abtrennt, zum Überstand eine Kalziumverbindung zusetzt und den Überstand klärt.

2. Verfahren nach Anspruch 1, dadurch gekennzeichnet, daß man eine Suspension von Zellen mit einem Gehalt an Trockenmaterial von 10 bis 25% und mit einem pH-Wert von 4,4 bis 6,8 bereitet und, zur Bewirkung der verstärkten Autolyse, nach mechanischer Desintegration der Zellen wenigstens ein bei neutralem pH-Wert aktives proteolytisches Enzym zusetzt, unter mäßiger Bewegung 15 bis 22 h bei neutralem pH-Wert inkubiert und während der Inkubation zu mehreren Malen insgesamt 300 bis 2000 Teile pro Million eines Sulfits und insgesamt 300 bis 2000 Teile pro Million Formaldehyd zusetzt.

3. Verfahren nach Anspruch 1, dadurch gekennzeichnet, daß man eine Suspension von Zellen mit einem Gehalt an Trockenmaterial von 10 bis 25% und mit einem pH-Wert von 3,0 bis 5,5 bereitet, 200 bis 2000 Teile pro Million eines Sulfits zusetzt und, zur Bewirkung der verstärkten Autolyse, nach mechanischer Desintegration der Zellen wenigstens ein bei saurem pH-Wert aktives Enzym zusetzt und unter mäßiger Bewegung 15 bis 22 h bei saurem pH-Wert inkubiert.

4. Verfahren nach Anspruch 1, dadurch gekennzeichnet, daß man nach der verstärkten Autolyse die Suspension 35 bis 70 min auf 90 bis 95°C erwärmt und die unlöslichen Anteile durch Zentrifugieren bei 60 bis 80°C abtrennt.

5. Verfahren nach Anspruch 1, dadurch gekennzeichnet, daß man die Kalziumverbindung in zum Sulfit äquimolarer Menge bei einem pH-Wert von 6,5 bis 7,5 zusetzt und durch Abtrennen des solcherart erhaltenen Niederschlags klärt.

6. Verfahren nach Anspruch 1, dadurch gekennzeichnet, daß das zugesetzte Enzym Papain ist, daß man es in einer Menge von wenigstens 0,3 g je l Suspension zusetzt und daß man bei einer Temperatur von 45 bis 65°C inkubiert.

## Claims

1. A process for the production of a yeast extract which comprises preparing a suspension of yeast cells, mechanically disintegrating the cells, subjecting the cells to autolysis, the autolysis being intensified by the addition of a proteolytic enzyme, and a sulphite being added to the yeast suspension before or after mechanical disintegration, separating insoluble fraction from the hydrolysed suspension, adding a compound of calcium to the supernatant liquid and clarifying the supernatant liquid.

2. A process as claimed in Claim 1, wherein, a suspension of cells having a dry matter content of from 10 to 25% and a pH value of from 4.4 to 6.8 ist prepared and, to carry out intensified autolysis after mechanical disintegration of the cells, at least one proteolytic enzyme active at a neutral pH is added, the whole ist incubated with gentle stirring for 15 to 22 h at a neutral pH and a total of from 300 to 2000 ppm of a sulphite and a total of from 300 to 2000 ppm of formaldehyde are added in several batches during the incubation process.

3. A process as claimed in Claim 1, wherein a suspension of cells having a dry matter content of from 10 to 25% and a pH value of from 3.0 to 5.5 is prepared, from 200 to 2000 ppm of a sulphite are added and, to carry out intensified autolysis after mechanical disintegration of the cells, at least one enzyme active at an acid pH is added and the whole is incubated with gentle stirring for 15 to 22 h at an acid pH.

4. A process as claimed in Claim 1, wherein, after the intensified autolysis step, the suspension ist heated for 35 to 70 minutes at 90 to 95°C and the insoluble fractions are separated by centrifuging at 60 to 80°C.

5. A process as claimed in Claim 1, wherein the compound of calcium is added in the same molar quantity as the sulphite at a pH of 6.5 to 7.5 and clarification ist effected by separating the precipitate thus obtained.

6. A process as claimed in any of Claim 1, wherein the enzyme added is papain, the papain is added in a quantity of at least 0.3 g per litre of suspension and the whole is incubated at a temperature of from 45 to 65°C.